# EUROPEAN PATENT APPLICATION

(11) **EP 0 576 050 A1**
(43) Date of publication of application: **29.12.1993**
(21) Application number: 93201177.8
(22) Date of filing: 23.04.1993
(51) Int. Cl.: C12N 15/31, C12N 15/70, C12N 15/75, C12N 1/20

(54) **Isolation and characterisation of the sfp gene of Bacillus subtilis**

(30) Priority: 24.04.1992 IT MI920976
(71) Applicant: ENIRICERCHE S.p.A., I-20121 Milan (IT)
(72) Inventor: Grandi, Guido, I-20090 San Felice-Segrate, Milan (IT); Cosmina, Paola, I-20146 Milan (IT); Rodriguez, Francesco, I-20097 San Donato Milanese, Milan (IT); Perego, Marta, I-23022 Chiavenna, Sondrio (IT); De Ferra, Francesca, I-20075 Lodi, Milan (IT)
(74) Representative: Fusina, Gerolamo

(57) **Abstract**

A description is given of the isolation and characterisation of the sfp gene in Bacillus subtilis which codes for a protein essential for producing the lipopeptide surfactin.

## Description

This invention relates to the isolation and characterisation of the sfp gene of Bacillus subtilis (B.subtilis) which codes for a protein essential for surfactin synthesis.

Surfactin is a cyclic lipopeptide produced from B.subtilis having the following sequence: 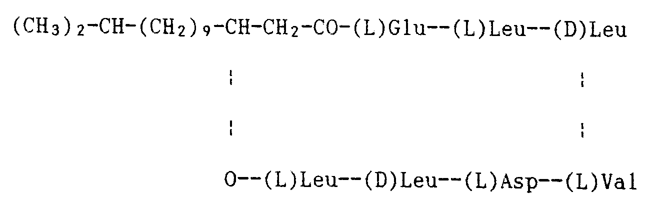
Surfactin is a powerful surfactant with anti-cholesterase, fungicidal and antibiotic properties. In addition, surfactin can be used as an anticoagulant in the prophylaxis of thrombosis, and generally for the prevention of illnesses such as myocardium infarct and pulmonary emboly, as it inhibits the fibrinogen-thrombin reaction by slowing fibrin formation.

Hence because of its multiple activity, surfactin is of particular interest as it finds wide application in the pharmaceutical, energy and environmental fields.

Currently available methods for producing surfactin are based essentially on fermentation of the strain B.subtilis ATCC 21332 isolated by Arima K. et al. (US 3,687,926) or its mutants [Mulligan C. et al. Appl. Microbiol. Biotech., 31: 486-489, (1989); IT-20738 A/90 and IT-22176 A/90].

However, none of these methods has proved sufficiently economical to allow its development on an industrial scale, mainly because of the low productivity of the currently available micro-organisms.

The application of molecular cloning to surfactin-producing micro-organisms could enable the productivity of this lipopeptide to be improved.

To allow such an approach, the gene or genes involved in surfactin synthesis have obviously to be isolated, sequenced and possibly modified.

Genetic and molecular biology studies conducted on the B.subtilis chromosome have identified three gene loci (sfp, srfA and comA) involved in surfactin biosynthesis, of which sfp and srfA map on the bacterial chromosome between the aroI (aromatic amino acid) and mtlB (use or mannitol) markers [Nakano, M.M. et al., (1988), J. Bacteriology 170: 5662-5668; Nakano, M.M. and Zuber P. (1989), J. Bacteriology 171: 5347-5353].

Specifically, the sfp locus is defined as the genetic determinant which differentiates the surfactin producer strain from the surfactin non-producer strains in which the srfA region, which codes for the multienzyme complex surfactin-synthetase which catalyzes the non-ribosomal polymerization of the lipopeptide surfactin, is integral.

The sequence characterisation of the chromosomal DNA from producer and non-producer strains of the sfp gene which codes for a protein essential in surfactin production is now reported for the first time.

Consequently one aspect of the present invention is a substantially pure DNA sequence isolated from the chromosomal DNA of Bacillus subtilis, where said DNA sequence codes for a protein essential for surfactin synthesis.

A further aspect of the present invention is a vector containing the DNA sequence which codes for a protein essential for surfactin synthesis.

A further aspect of the present invention is a micro-organism transformed by a vector containing the DNA sequence which codes for a protein essential for surfactin synthesis.

A further aspect of the present invention is a method for producing surfactin by means of a culture of host cells transformed with a vector containing the DNA sequence which codes for a protein essential for surfactin synthesis.

A further aspect of the present invention is the use of the substantially pure DNA sequence isolated from the chromosomal DNA of Bacillus subtilis, for the in vitro or in vivo production of surfactin analogues.

Further aspects of the present invention will be apparent from the ensuing description.

### Description of the figures:

Figure 1: this shows the restriction map of that chromosomal DNA region containing the sfp gene; the vertical arrow indicates the point of insertion of the Tn917 transposon of the chromosomal DNA of the JH642::Tn8 clone.

Fire 2: this shows the nucleotide sequence of the sfp gene isolated from a surfactin producer strain.

Fire 3: this shows the nucleotide sequence of the sfp gene isolated from a surfactin non-producer strain.

Figure 4: this shows the amino acid sequence of the protein coded by the sfp gene isolated from a surfactin producer strain.

Figure 5: this shows the amino acid sequence of the protein coded by the sfp gene isolated from a surfactin non-producer strain.

Figure 6: this shows a comparison between the amino acid sequences of the proteins coded by the sfp gene isolated from a surfactin producer strain and, respectively, that isolated from a surfactin non-producer strain.

### Detailed description of the invention

The cloning strategy was based on assays of surfactin production or non-production after transforming suitable B.subtilis strains with chromosomal DNA.

A gene bank was constructed by randomly integrating the Tn917 transposon, containing the gene which codes for erythromycin resistance, into the chromosome of a non-producer strain such as B.subtilis JH642 (phe⁻, trp⁻) (M.M. Nakano and P. Zuber (1989), J. Bacteriol., 171: 5347-5353).

The chromosomal DNA isolated from the erythromycin-resistant clones forming part of the gene bank was then used to transform cells of the surfactin producer strain B.subtilis JH642srf4, selected for resistance to erythromycin.

Finally, from the erythromycin-resistant colonies those no longer able to produce surfactin (srf⁻) were selected by the method described by Cooper, D.G.et al (appearance of haloes about srf⁺ colonies) (1981, Appl. Environm. Microbiol., 42: 408-412).

Genetic mapping, effected by transduction with the PBS-1 bacteriophage (Hoch, J.A. et al. (1967) J. Bacteriology 93: 1925-1937) showed that some of the integrations into the Erm^{R}, srf⁻ colonies mapped between the aroI and mtlB markers.

The Erm^{R}, srf⁻ character of these clones could be acquired by at least two different integration phenomena.

In a first type of integration the transposon is inserted into the srfA locus, the srf⁻ phenotype being due to the interruption of the srfA locus.

Alternatively, integration can take place outside the srfA and can involve replacing the sfp locus of the receiving strain (which determines surfactin production) with the negative surfactin locus of the donor strain. To distinguish between the two phenomena, the DNAs isolated from the Erm^{R}, srf⁻ colonies were used to retransform cells of the producer strain B.subtilis JH642srf4. It was hence observed that the DNA of one of said colonies transformed the producer strain both into Erm⁺, srf⁺ colonies and into Erm⁻, srf⁺ and Erm⁺, srf⁻ colonies, whereas all the others transformed said strain into Erm⁺, srf⁻ colonies. These results indicated that in the colony denominated JH642::Tn8 the transposon was not inserted into the srfA locus but was adjacent to sfp.

The chromosomal DNA adjacent to Tn917 from JH642::Tn8 was cloned by the method described by Youngman et al. [(1984), Mol. Gen. Genet. 195: 424-433] by using the pTV21 and pTV20 plasmids.

In this manner it was possible to isolate a plasmid (pTn2000) comprising a chromosomal fragment containing the sfp locus.

In order to isolate the minimum sequence responsible for the surfactin non-producer character, the DNA fragment isolated from the pTn2000 plasmid was digested with suitable restriction enzymes, then cloned into a plasmid integration vector chosen from pJM7145, pJM7146, pJM103 and pJM 102 and used to transform the surfactin producer strain B.subtilis JH642srf4.

The choice of this strategy is based on the principle that B.subtilis integration vectors replicate in Escherichia coli (**E,coli**), but are not able to replicate in B.subtilis as they do not contain that DNA portion responsible for replication of the plasmid in this micro-organism. These plasmid vectors are however able to integrate into the B.subtilis chromosome via homologous recombination and hence express the resistance marker which they contain. Following integration of the plasmid, the chromosomal sequence is interrupted to hence lead to possible inactivation of the genes in which integration has occurred.

This mechanism can occur only if there is a B.subtilis chromosomal DNA fragment on the plasmid to provide the homology region necessary for crossing-over and hence integration to take place.

Analysis of the transformants has shown that the minimum DNA fragment which transformed this producer strain into colonies with srf⁻ phenotype was a HindIII-HindIII fragment of about 800 bp contained in the plasmid denominated pSRF2007.

To isolate the DNA containing the sfp gene from both producer and non-producer strains, the DNA fragment contained in the pSRF2007 plasmid was used as a probe for selecting some positive clones from a chromosomal DNA bank of the non-producer strain B.subtilis JH642 constructed in the lambda FixII phage (Stratagene Inc.) after hybridization by the southern technique (T. Maniatis et al. "Molecular cloning: a laboratory manual" Cold Spring Harbor Laboratory, 1982).

Operating in this manner, some positive lambda clones were identified having a similar restriction map and containing a SacI fragment of about 4 Kb which hybridized to the pSRF2007 fragment labelled with ³²P.

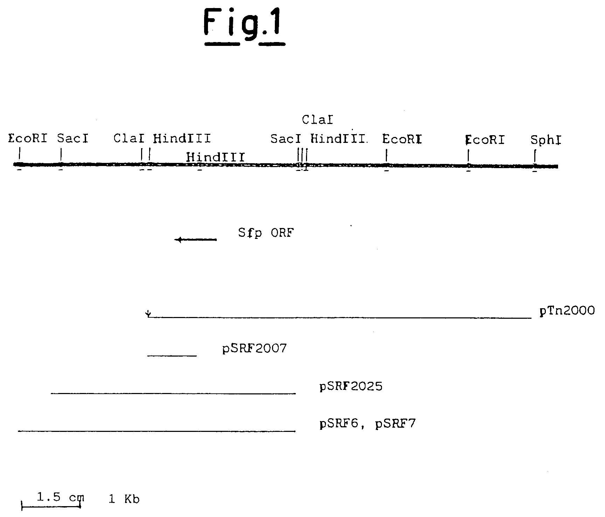

This fragment was subcloned in an integration vector, resulting in the construction of the pSRF2025, plasmid (Figure 1).

By transforming the producer strain B.subtilis JH642srf4 with pSRF2025, colonies with both srf⁺ phenotype and srf⁻ phenotype were obtained, depending on the point of insertion of the plasmid into the B.subtilis chromosomal DNA.

To clone the sfp allele in the srf⁺ phenotype, the chromosomal DNA was extracted both from an srf⁺ colony and from an srf⁻ colony obtained after transforming B.subtilis JH642srf4 with pSRF2025, then digested with a restriction enzyme chosen so as not to have an insertion site within the integration plasmid, and finally linked to itself in the presence of T4 DNA ligase.

The ligase mixtures were then used to transform E.coli competent cells, selected for resistance to an antibiotic the gene of which is contained in the integration plasmid.

The positive clones were those which contained the plasmids formed from the integration plasmid and a portion of B.subtilis chromosomal DNA. These plasmids were able to circularize during the ligase reaction.

The plasmid isolated from an srf⁺ colony was denominated pSRF6, whereas that isolated from an srf⁻ colony was denominated pSRF7.

On the basis of the method used for this cloning, pSRF7 had to contain the surfactin non-producer wild type allele and could therefore transform the B.subtilis JH642srf4 strain into a strain with srf⁻ phenotype. In contrast, the pSRF6 plasmid had to contain the producer allele and hence after transformation of the non-producer strain B.subtilis JH642 could give it the capacity to produce surfactin by integration (srf⁺ phenotype). Genetic analysis by transformation confirmed the expected results.

The pSRF6 and pSRF7 plasmids were then sequenced by the method of Sanger, F. and Coulson, A. (1975) (J. MOl. Biol., 94: 441-449) in the region of interest, corresponding to the pSRF2007 insertion, and in the regions adjacent to it.

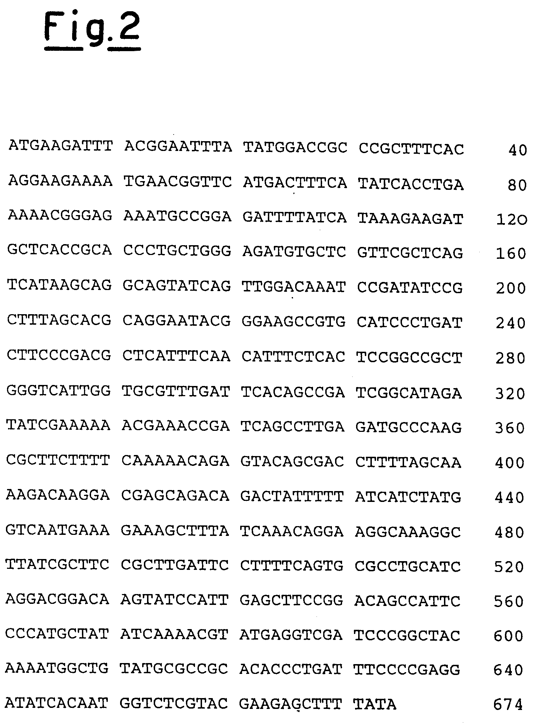

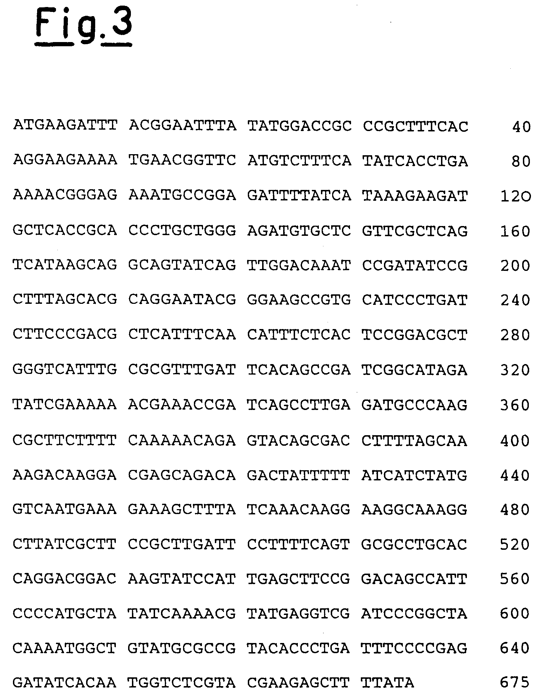

Analysis of the sequence of that chromosomal region identified genetically as essential for surfactin production showed that the sfp gene isolated from a non-producer strain differs from the corresponding gene isolated from a producer strain (Figure 2) by the insertion of an adenine (A) in position 468 (Figure 3).

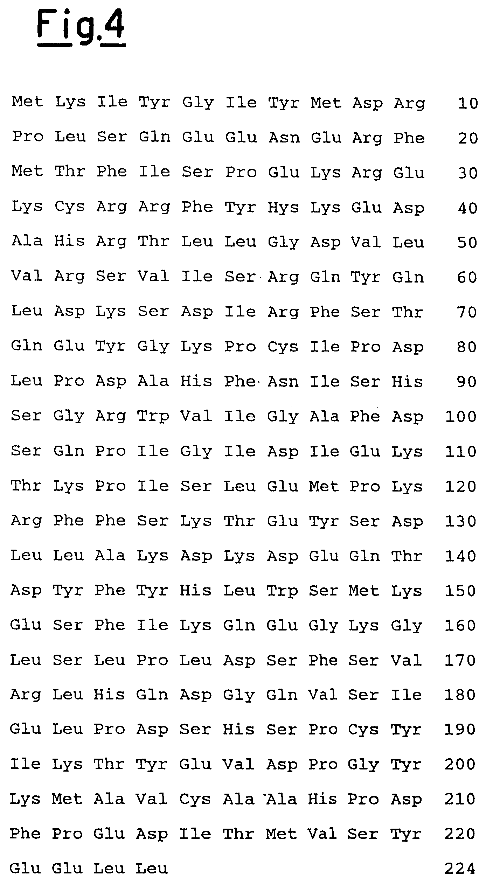

The sfp gene isolated from the producer strain codes for a protein of 224 amino acids the sequence of which is shown in Figure 4.

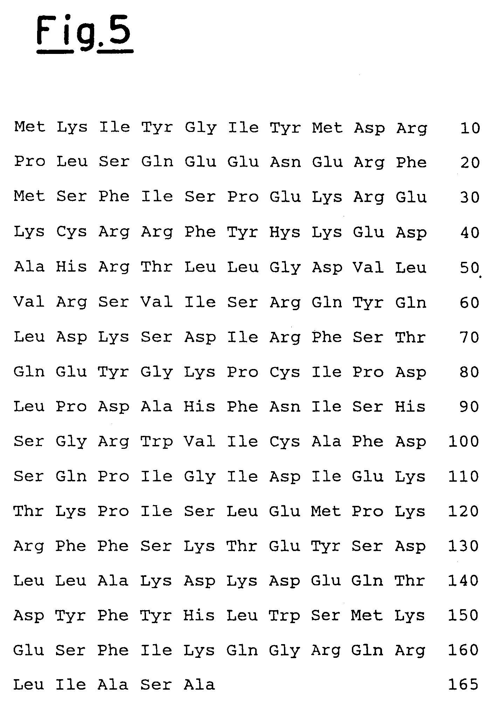

The nucleotide sequence difference leads to the synthesis of the non-producer strain of a non-functional truncated protein (165 amino acids) (Figure 5).

The availability of these sequences enables the surfactin production levels to be modified by, for example, overproduction of the sfp gene product in a strain which overproduces surfA. In addition, the sfp nucleotide sequence can be used to modify the phenotype of a surfactin non-producer strain.

Tile sfp gene sequence, or the product of its gene expression, can also be used for in vitro or in vivo production of peptides similar to surfactin.

The expression of the 224 amino acid protein coded by the sfp gene can be obtained by cultivating in a culture medium containing a nitrogen source, a carbon source and microelements, a host micro-organism transformed with an expression vector containing said gene positioned downstream of expression regulator sequences (promoter, RBS and terminator).

Vectors suitable for the purpose can be chosen from plasmids, phages and cosmids available commercially and from collection centres.

The pSMSRF6 plasmid was filed in accordance with the Treaty of Budapest at the American Type Culture Collection where it received the filing number ATCC 68913.

The purpose of the following examples is merely to describe the present invention in greater detail, and must in no way be interpreted as limitative of the scope of the present invention.

### EXAMPLE 1

### Isolation and cloning of the sfp gene

A) Construction of an insertion gene bank from B.subtilis JH642 chromosomal DNA with the Tn917 transposon
   The pTV5TS plasmid DNA containing the Tn917 transposon (1 µg) was used to transform competent cells of B.subtilis JH642 (surfactin non-producer) by the method described by Contente and Dubnau [Mol. Gen. Genetics (1979), 167: 251-258].
   The transformants were selected by plating the cells on 20 ml of maximum medium VY (veal infusion) [veal infusion broth (DIFCO) 25 g/l, yeast extract 5 g/l and agar (DIFCO) 20 g/l] and pouring onto the plates 5 ml of soft agar containing 125 µg/ml of erythromycin then incubating the plates at 37°C for a further 18 hours.
   The chromosomal DNA extracted from the erythromycin-resistant transformants (Erm^{R}), ie those in which homologous recombination had taken place at the chromosomal level, was used to transform competent cells of the producer strain B.subtilis JH642srf4.
   The transformed colonies were selected for resistance to erythromycin in the aforestated manner.
   Finally, the Erm-resistant colonies were transferred onto plates containing blood and selected for appearance of hemolysis haloes as an indicator of surfactin production [Cooper, D.G. et al., Appl. Environm. Microbiol., 42: 408-412 (1981)].
   Nine surfactin non-producing colonies were isolated (srf⁻ phenotype).
B) Mapping by transduction with the PBS-1 phage
   The purpose of the experiment was to locate the DNA portion responsible for the loss of the surfactin production character.
   Genetic mapping was effected by transduction with the PBS-1 bacteriophage [Hoch, J.A. et al. (1967) J. Bacteriology, 93: 1925-1937).
   a) Preparation of PBS-1 lysate
      The lysate of the PBS-1 phage was prepared on the srf⁻ colonies. 20 ml volumes of VY culture medium [25 g/l veal infusion broth, 5 g/l yeast extract (DIFCO)] were each inoculated with 0.5 ml of an overnight culture of the srf⁻ colonies. The cultures were followed until termination of the logarithmic growth phase, and then 1 ml was diluted in 9 ml of Y medium (NaCl 4 g/l, K₂SO₄ 5 g/l, KH₂PO₄ 1.5 g/l, Na₂HPO₄ 3 g/l, MgSO₄.7H₂O 0.12 g/l, CaCl₂ 0.01 g/l, FeCl₂ 0.01 g/l, yeast extract 1 g/l, pH 7.0).
      An aliquot (0.5 ml) of the bacterial suspension was infected with 1 ml of a 10⁻⁶ dilution of PBS-1 lysate in Y medium.
      The mixture was incubated for 20 minutes at 37°C without agitation, 2 ml of TBAB soft agar (DIFCO) were added (prepared by diluting TBAB in sterile water in a 65:40 w/v ratio), poured onto TBAB plates and incubated overnight at 37°C.
      The following morning 2 turbid plaques were withdrawn from the plate and inoculated into 40 ml of VY medium. After 2 hours of incubation at 37°C with agitation, the culture was incubated at 37°C overnight without agitation.
      The lysate obtained in this manner was centrifuged for 10 minutes at 10,000 rpm. Dnasi (Worthington) and MgCl₂ were then added to the supernatant to a final concentration of 25 µg/ml and 0.05 M respectively. After 30 minutes of incubation at 37°C the lysate was sterilized by filtration using Millipore 0.45 µm filters.
   b) Transduction of B.subtilis PB5069 with the lysate PBS-1
      The B.subtilis PB5069 strain [M. Perego and J. Hoch (1991), J. Bacteriol. 173: 2514-2520], auxotrophic by the markers aroI (requirement for aromatic amino acids) and mtlB (inability to use mannitol as carbon source) was transduced with the PBS-1 lysate prepared on the donor strain (D) JH642-4 sfr⁺.
      In practice, a culture of the B.subtilis PB5069 receiving strain was inoculated into 10 ml of PY medium, and the growth followed under the microscope until the end of the logarithmic phase and the attainment of bacterial mobility.
      Aliquots of the culture (0.5 ml) were then infected with 100 µl of the lysate PBS-1 prepared as under point a).
      The phage was adsorbed onto the cells for 20 minutes at 37°C. After initial centrifuging (5 minutes at 7000 rpm), the cells were washed with 0.5 ml of MT minimum transformation medium, re-centrifuged, resuspended in 100 µl of MT medium and selected by each of the auxotroph markers.
      This selection was effected by plating the cells on MT minimum medium containing 4% glucose and all the amino acids required by the strain except that for which the selection is made, to a final concentration of 25 µg/ml. Operating in this manner, 6 colonies mapping between the aroI and mtlB markers were selected.
C) To determine whether the loss of surfactin production character in these colonies was due to the integration of a portion of DNA containing the Tn917 transposon in the srfA gene (surfactin synthetase) or due to the integration of a chromosomal DNA portion containing both the Tn917 transposon and a region adjacent to it comprising the sfp character (non-producer wild type allele) of the original strain of the insertion gene bank, the following experiment was carried out.
   Chromosomal DNA (100 ng) isolated from each of the six colonies was used to transform cells of the producer strain B.subtilis JH642srf4. The transformants were then selected for resistance to erythromycin and for surfactin production in the aforestated manner.
   The results showed that the chromosomal DNA of only one colony (JH642srf::Tn8) allowed transformation of the producer strain both into Erm⁺ srf⁻, and into Erm⁻ srf⁻ and Erm⁺ srf⁻, whereas all the other DNAs transformed the producer strain into Erm⁺ srf⁻. This indicated that in said colony the transposon had been inserted not into the srfA locus, but was adjacent to the sfp locus isolated from the gene bank of the non-producer strain.
D) Isolation of the pTn2000 plasmid
   The chromosomal DNA adjacent to Tn917 from JH642::Tn8 was cloned by the method described by Youngman et al. (1984), (Mol. Gen. Genet. 195: 424-433) by using the pTV21 and pTV20 plasmids which contain sequences homologous to Tn917 and an origin of replication in E.coli.
   The pTV21 and pTV20 plasmid DNAs (100 ng) were linearized with XbaI (BIOLABS) and used to transform 200 µl of B.subtilis JH642::Tn8. The transformants were then selected for resistance to chloramphenicol (Cm^{R}) and sensitivity to erythromycin (indicating that double crossing-over has occurred).
   The chromosomal DNA was isolated from the Cm^{R} colonies by the method of Marmur (1961) (J. Mol. Biol., 3: 208-218).
   An aliquot (1 µg) of said DNA was completely digested by the enzyme SphI, and after extraction with phenol and precipitation with ethanol was resuspended in ligation buffer at a concentration of 5 µg/ml in a final volume of 200 µl containing 20 units of T4 DNA ligase.
   After incubating at 37°C for about 12 hours, the ligation mixture was used to transform competent E.coli DH5α cells (BRL). The transformants were selected for resistance to chloramphenicol.
   From a Cm^{R} colony the pTn2000 plasmid was isolated, containing an insert of about 7 kilobases adjacent to the transposon on the side of the sfp gene (Figure 1). It was not possible to isolate any clone on the other side. The position of the pTn2000 insert relative to the Tn917 transposon and the srfA operon was verified by the southern blot technique on chromosomal DNA using as probe srfA inserts and the pTn2007 insert.
E) Identification of the minimum region responsible for the srf⁻ character
   An aliquot (1 µg) of chromosomal DNA isolated from the Tn2000 plasmid was digested totally for 1 hour at 37°C with HindIII restriction enzyme (Biolabs).
   On completion of the digestion, the DNA was purified with one volume of phenol, one volume of phenol/choroform (1:1 v/v), and 1 volume of chloroform. The DNA was then precipitated with 2 volumes of ethanol (99%) in 0.5 M Tris-HCl pH 8.0 for 10 minutes at -80°C. After centrifuging for 10 minutes at 12,000 rpm in a microcentrifuge (Eppendorf ), the DNA was dried in a lyophilizer and then resuspended in 20 µl of sterile H₂O.
   One aliquot (6 µl) of the suspension was then ligated in a ligation mixture with the pJM103 integration plasmid [M. Perego and J. Hoch (1991), J. Bacteriol. 173: 2514-2520], (200 ng), previously linearized with the HindIII restriction enzyme.
   The resultant ligation mixture, containing 1 unit of the enzyme T4-DNA ligase, was incubated for 16 hours at 14°C.
   1 µl of a 1/10 dilution of said mixture was used to transform 100 µl of E.coli JM109 competent cells (BRL).
   The transformants were plated on LB plates containing chloramphenicol (100 µg/ml), IPTG (isopropyl-β-D-thiogalacto-pyranoside) (0.5 mM) and X-Gal (5-bromo-4-chloro-3-indolyl-D-galactopyranoside( (20 µg/ml).
   The plasmid DNA was extracted from transformant colonies having a white coloration, indicating a DNA fragment in the pJM103 vector, by the fast extraction method described by Holmes and Quigley ((1981) Anal. Biochem., 114: 193-197), and the plasmids were divided into groups which differed from each other by the size of the cloned DNA fragment.
   The plasmid DNA of each group was used to transform cells of the producer strain B.subtilis JH642srf4.
   The transformants were then selected on Shaeffer medium with 5 µg/ml of chloramphenicol added.
   The transformants which were chloramphenicol-resistant, indicating integration of the plasmid DNA, were replicated on selective TBAB plates with an added 5% of defibrinated ram's blood and assayed for disappearance of the hemolysis halo.
   The results showed that integration of one of the fragments deriving from the pTn2000 plasmid resulted in inactivation of the surfactin production character.
   The plasmid containing said HindIII-HindIII fragment of about 800 bases was denominated pSRF2007.
F) Construction of the gene bank in the lambda FixII phage
   To isolate the DNA containing the sfp gene from producer and non-producer strains, the DNA fragment contained in the pSRF2007 plasmid labelled with ³²P was used as probe for selecting a chromosomal DNA bank of the non-producer strain B.subtilis JH642 constructed in the lambda FixII phage (STRATAGENE Inc.) by the method described in T. Maniatis et al. "Molecular cloning: a laboratory manual" Cold Spring Harbor Laboratory 1982.
   Screening identified 9 positive lambda clones with a similar restriction map and containing an SacI fragment of about 4 Kb which hybridized at the ³²P-labelled pSRF2007 insert.
   Said fragment was subcloned in pJM103, resulting in the construction of the pSRF2025 plasmid (Figure 1).
   By transforming the producer strain B. subtilis JH642-4 srf⁺ with pSRF2025 both colonies with srf⁺ phenotype and colonies with srf⁻ phenotype are obtained depending on the point at which the plasmid is inserted into the B.subtilis chromosomal DNA.
   To clone the sfp allele which appears in the srf⁺ phenotype, the chromosomal DNA was extracted both from an srf⁺ colony and from an srf⁻ colony obtained as stated.
   An aliquot (1 µg) of each chromosomal DNA was completely digested for 1 hour at 37°C with the restriction enzyme EcoRI (Biolabs).
   On completion of digestion, the DNAs were purified with one volume of phenol, one volume of phenol/choroform (1:1 v/v), and 1 volume of chloroform. The DNAs were then precipitated with 2 volumes of ethanol (99%) in 0.5 M Tris-HCl pH 8.0 for 10 minutes at -80°C.
   After centrifuging for 10 minutes at 12,000 rpm in a microcentrifuge (Eppendorf), the DNAs were dried in a lyophilizer and then resuspended in 20 µl of sterile H₂O.
   One aliquot (6 µl) of each suspension was then incubated in a ligation mixture in the presence of T4 ligase at 12°C for 16 hours to recirculate the plasmid.
   The ligase mixtures were then used to transform E.coli DH5α competent cells subsequently selected for resistance to ampicillin (Amp).
   The plasmid DNAs were then isolated from the Amp-resistant colonies.
   The plasmid obtained from the srf⁺ colony was denominated pSRF6, whereas that obtained from the srf⁻ colony was denominated pSRF7.
   The plasmids where then used to transform by integration the surfactin non-producer strain B.subtilis JH642 and the producer strain B.subtilis JH642srf respectively. The transformants were assayed for surfactin production.
   The results showed that on transforming JH642, srf⁺ colonies were also obtained, whereas on transforming JH642srf, srf⁻ colonies were also obtained. This confirmed that pSRF6 contained the functional sfp allele, whereas pSRF7 contained the non-functional allele isolated from the non-producer strain.

### EXAMPLE 2

### Sequencing of the sfp gene

The pSRF6 and pSRF7 plasmids containing the sfp gene from chromosomal DNAs of non-producer and producer strains respectively were sequenced in the region of interest, corresponding to the HindIII-HindIII insert of about 800 bases of pSRF2007 and the regions adjacent to it, by the method of Sanger, F. and Coulson, A. (1975) (J. MOl. Biol., 94: 441-449).

The sfp gene nucleotide sequences isolated from pSRF6 and pSRF7 are shown in Figure 2 and Figure 3 respectively.

The sfp gene in the producer strain potentially codes for a protein of 224 amino acids (from the first methionine in the open reading phase) with a molecular weight of 26.2 kilodaltons (Figure 4).

The insertion of the "non-producer" sfp allele of an adenine in position 468 (see Figure 3) changes the reading phase downstream of the sfp insertion in the C-terminal part of the product, and results in the production of a protein of 165 amino acids (from the first methionine) with a molecular weight of 19.6 kilodaltons and with the sequence shown in Figure 5. It is therefore deduced that the sfp allele with this sequence is not functional.

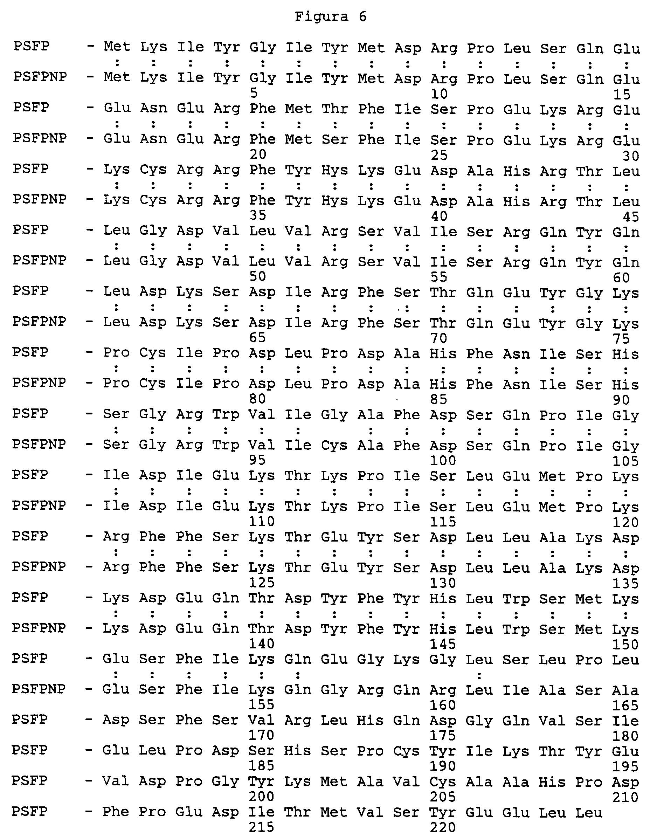

Figure 6 shows the comparison between the amino acid sequences of the sfp alleles from non-producer and producer strains. From this figure it can be seen that the amino acid sequences of said alleles also differ in positions 22 and 97 of the amino acid sequence.

The functional studies reported in Example 1 indicate that the insertion of an A in position 468 of the nucleotide sequence is the determining factor of the phenotype, as it is the only mutation which falls within the HindIII-HindIII region of about 800 bases contained in the pSRF2007 plasmid.

SEQUENCE LISTING

(1) GENERAL INFORMATION:

(i) APPLICANT:
(A) NAME: ENIRICERCHE S.p.A.
(B) STREET: VIA F. MARITANO, 26
(C) CITY: S. DONATO MILANESE, (MILANO)
(E) COUNTRY: ITALY
(F) POSTAL CODE: 20097

(ii) TITLE OF INVENTION:
ISOLATION AND CHARACTERIZATION OF SFP GENE OF BACILLUS
SUBTILIS

(iii) NUMBER OF SEQUENCES: 4

(iv) COMPUTER READABLE FORM:
(A) MEDIUM TYPE: Diskette
(B) COMPUTER: IBM PC compatible
(C) OPERATING SYSTEM: PC-DOS/MS-DOS
(D) SOFTWARE: IBM DISPLAY WRITER VERSION 1.5

(1) INFORMATION FOR SEQ ID No: 1

(i) SEQUENCE CHARACTERISTICS

(A) LENGTH: 674 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION:

SEQ ID NO:1:

ATGAAGATTT ACGGAATTTA TATGGACCGC CCGCTTTCAC AGGAAGAAAA 50
TGAACGGTTC ATGACTTTCA TATCACCTGA AAAACGGGAG AAATGCCGGA 100
GATTTTATCA TAAAGAAGAT GCTCACCGCA CCCTGCTGGG AGATGTGCTC 150
GTTCGCTCAG TCATAAGCAG GCAGTATCAG TTGGACAAAT CCGATATCCG 200
CTTTAGCACG CAGGAATACG GGAAGCCGTG CATCCCTGAT CTTCCCGACG 250
CTCATTTCAA CATTTCTCAC TCCGGCCGCT GGGTCATTGG TGCGTTTGAT 300
TCACAGCCGA TCGGCATAGA TATCGAAAAA ACGAAACCGA TCAGCCTTGA 350
GATGCCCAAG CGCTTCTTTT CAAAAACAGA GTACAGCGAC CTTTTAGCAA 400
AAGACAAGGA CGAGCAGACA GACTATTTTT ATCATCTATG GTCAATGAAA 450
GAAAGCTTTA TCAAACAGGA AGGCAAAGGC TTATCGCTTC CGCTTGATTC 500
CTTTTCAGTG CGCCTGCATC AGGACGGACA AGTATCCATT GAGCTTCCGG 550
ACAGCCATTC CCCATGCTAT ATCAAAACGT ATGAGGTCGA TCCCGGCTAC 600
AAAATGGCTG TATGCGCCGC ACACCCTGAT TTCCCCGAGG ATATCACAAT 650
GGTCTCGTAC GAAGAGCTTT TATA 674

(2) INFORMATION FOR SEQ ID NO:2:

(i) SEQUENCE CHARACTERISTICS

(A) LENGTH: 675 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION:
SEQ ID NO:2:

ATGAAGATTT ACGGAATTTA TATGGACCGC CCGCTTTCAC AGGAAGAAAA 50
TGAACGGTTC ATGTCTTTCA TATCACCTGA AAAACGGGAG AAATGCCGGA 100
GATTTTATCA TAAAGAAGAT GCTCACCGCA CCCTGCTGGG AGATGTGCTC 150
GTTCGCTCAG TCATAAGCAG GCAGTATCAG TTGGACAAAT CCGATATCCG 200

CTTTAGCACG CAGGAATACG GGAAGCCGTG CATCCCTGAT CTTCCCGACG 250
CTCATTTCAA CATTTCTCAC TCCGGACGCT GGGTCATTTG CGCGTTTGAT 300
TCACAGCCGA TCGGCATAGA TATCGAAAAA ACGAAACCGA TCAGCCTTGA 350
GATGCCCAAG CGCTTCTTTT CAAAAACAGA GTACAGCGAC CTTTTAGCAA 400
AAGACAAGGA CGAGCAGACA GACTATTTTT ATCATCTATG GTCAATGAAA 450
GAAAGCTTTA TCAAACAAGG AAGGCAAAGG CTTATCGCTT CCGCTTGATT 500
CCTTTTCAGT GCGCCTGCAC CAGGACGGAC AAGTATCCAT TGAGCTTCCG 550
GACAGCCATT CCCCATGCTA TATCAAAACG TATGAGGTCG ATCCCGGCTA 600
CAAAATGGCT GTATGCGCCG TACACCCTGA TTTCCCCGAG GATATCACAA 650
TGGTCTCGTA CGAAGAGCTT TTATA 675

(3) INFORMATION FOR SEQ ID NO:3:

(i) SEQUENCE CHARACTERISTICS

(A) LENGTH: 224 amino acids
(B) TYPE: amino acid
(C) STRANDEDNESS: single
(D) TOPOLOGY: linear

(ii) MOLECULE TYPE: protein

(xi) SEQUENCE DESCRIPTION:
SEQ ID NO:3:

Met Lys Ile Tyr Gly Ile Tyr Met Asp Arg Pro Leu Ser Gln Glu
                5                   10                  15
Glu Asn Glu Arg Phe Met Thr Phe Ile Ser Pro Glu Lys Arg Glu
                20                  25                  30
Lys Cys Arg Arg Phe Tyr His Lys Glu Asp Ala His Arg Thr Leu
                35                  40                  45
Leu Gly Asp Val Leu Val Arg Ser Val Ile Ser Arg Gln Tyr Gln
                50                  55                  60
Leu Asp Lys Ser Asp Ile Arg Phe Ser Thr Gln Glu Tyr Gly Lys
                65                  70                  75
Pro Cys Ile Pro Asp Leu Pro Asp Ala His Phe Asn Ile Ser His

                80                  85                  90
Ser Gly Arg Trp Val Ile Gly Ala Phe Asp Ser Gln Pro Ile Gly
                95                  100                 105
Ile Asp Ile Glu Lys Thr Lys Pro Ile Ser Leu Glu Met Pro Lys
                110                 115                 120
Arg Phe Phe Ser Lys Thr Glu Tyr Ser Asp Leu Leu Ala Lys Asp
                125                 130                 135
Lys Asp Glu Gln Thr Asp Tyr Phe Tyr His Leu Trp Ser Met Lys
                140                 145                 150
Glu Ser Phe Ile Lys Gln Glu Gly Lys Gly Leu Ser Leu Pro Leu
                155                 160                 165
Asp Ser Phe Ser Val Arg Leu His Gln Asp Gly Gln Val Ser Ile
                170                 175                 180
Glu Leu Pro Asp Ser His Ser Pro Cys Tyr Ile Lys Thr Tyr Glu
                185                 190                 195
Val Asp Pro Gly Tyr Lys Met Ala Val Cys Ala Ala His Pro Asp
                200                 205                 210
Phe Pro Glu Asp Ile Thr Met Val Ser Tyr Glu Glu Leu Leu
                215                 220

(4) INFORMATION FOR SEQ ID NO:4:

(i) SEQUENCE CHARACTERISTICS:
(A) LENGTH: 165 amino acids
(B) TYPE: amino acid
(C) STRANDEDNESS: single
(D) TOPOLOGY: linear

(ii) MOLECULE TYPE: protein

(xi) SEQUENCE DESCRIPTION:
SEQ ID NO:4:

Met Lys Ile Tyr Gly Ile Tyr Met Asp Arg Pro Leu Ser Gln Glu
                5                   10                  15
Glu Asn Glu Arg Phe Met Ser Phe Ile Ser Pro Glu Lys Arg Glu

                20                  25                  30
Lys Cys Arg Arg Phe Tyr His Lys Glu Asp Ala His Arg Thr Leu
                35                  40                  45
Leu Gly Asp Val Leu Val Arg Ser Val Ile Ser Arg Gln Tyr Gln
                50                  55                  60
Leu Asp Lys Ser Asp Ile Arg Phe Ser Thr Gln Glu Tyr Gly Lys
                65                  70                  75
Pro Cys Ile Pro Asp Leu Pro Asp Ala His Phe Asn Ile Ser His
                80                  85                  90
Ser Gly Arg Trp Val Ile Cys Ala Phe Asp Ser Gln Pro Ile Gly
                95                  100                 105
Ile Asp Ile Glu Lys Thr Lys Pro Ile Ser Leu Glu Met Pro Lys
                110                 115                 120
Arg Phe Phe Ser Lys Thr Glu Tyr Ser Asp Leu Leu Ala Lys Asp
                125                 130                 135
Lys Asp Glu Gln Thr Asp Tyr Phe Tyr His Leu Trp Ser Met Lys
                140                 145                 150
Glu Ser Phe Ile Lys Gln Gly Arg Gln Arg Leu Ile Ala Ser Ala
                155                 160                 165

## Claims

1. A substantially pure DNA sequence isolated from the chromosomal DNA of Bacillus subtilis, said DNA sequence coding for a protein essential for the synthesis of surfactin, wherein said DNA sequence has the following nucleotide sequence:

ATGAAGATTT ACGGAATTTA TATGGACCGC 30
CCGCTTTCAC AGGAAGAAAA TGAACGGTTC 60
ATGACTTTCA TATCACCTGA AAAACGGGAG 90
AAATGCCGGA GATTTTATCA TAAAGAAGAT 120
GCTCACCGCA CCCTGCTGGG AGATGTGCTC 150
GTTCGCTCAG TCATAAGCAG GCAGTATCAG 180
TTGGACAAAT CCGATATCCG CTTTAGCACG 210
CAGGAATACG GGAAGCCGTG CATCCCTGAT 240
CTTCCCGACG CTCATTTCAA CATTTCTCAC 270
TCCGGCCGCT GGGTCATTGG TGCGTTTGAT 300
TCACAGCCGA TCGGCATAGA TATCGAAAAA 330
ACGAAACCGA TCAGCCTTGA GATGCCCAAG 360
CGCTTCTTTT CAAAAACAGA GTACAGCGAC 390
CTTTTAGCAA AAGACAAGGA CGAGCAGACA 420
GACTATTTTT ATCATCTATG GTCAATGAAA 450
GAAAGCTTTA TCAAACAGGA AGGCAAAGGC 480
TTATCGCTTC CGCTTGATTC CTTTTCAGTG 520
CGCCTGCATC AGGACGGACA AGTATCCATT 540
GAGCTTCCGG ACAGCCATTC CCCATGCTAT 570
ATCAAAACGT ATGAGGTCGA TCCCGGCTAC 600
AAAATGGCTG TATGCGCCGC ACACCCTGAT 630
TTCCCCGAGG ATATCACAAT GGTCTCGTAC 660

GAGGAGCTTT TATA 674

2. The substantially pure DNA sequence as defined in claim 1, wherein said protein essential for surfactin synthesis and encoded by said DNA sequence has the following amino acid sequence:

Met Lys Ile Tyr Gly Ile Tyr Met Asp Arg 10
Pro Leu Ser Gln Glu Glu Asn Glu Arg Phe 20
Met Thr Phe Ile Ser Pro Glu Lys Arg Glu 30
Lys Cys Arg Arg Phe Tyr Hys Lys Glu Asp 40
Ala His Arg Thr Leu Leu Gly Asp Val Leu 50
Val Arg Ser Val Ile Ser Arg Gln Tyr Gln 60
Leu Asp Lys Ser Asp Ile Arg Phe Ser Thr 70
Gln Glu Tyr Gly Lys Pro Cys Ile Pro Asp 80
Leu Pro Asp Ala His Phe Asn Ile Ser His 90
Ser Gly Arg Trp Val Ile Gly Ala Phe Asp 100
Ser Gln Pro Ile Gly Ile Asp Ile Glu Lys 110
Thr Lys Pro Ile Ser Leu Glu Met Pro Lys 120
Arg Phe Phe Ser Lys Thr Glu Tyr Ser Asp 130
Leu Leu Ala Lys Asp Lys Asp Glu Gln Thr 140
Asp Tyr Phe Tyr His Leu Trp Ser Met Lys 150
Glu Ser Phe Ile Lys Gln Glu Gly Lys Gly 160
Leu Ser Leu Pro Leu Asp Ser Phe Ser Val 170
Arg Leu His Gln Asp Gly Gln Val Ser Ile 180
Glu Leu Pro Asp Ser His Ser Pro Cys Tyr 190
Ile Lys Thr Tyr Glu Val Asp Pro Gly Tyr 200
Lys Met Ala Val Cys Ala Ala His Pro Asp 210
Phe Pro Glu Asp Ile Thr Met Val Ser Tyr 220
Glu Glu Leu Leu 224

3. A plasmid comprising the DNA sequence claimed in claim 1.

4. The plasmid of claim 3, where said plasmid is a bacteria expression vector.

5. The plasmid of claim 3, where said plasmid is pSMSRF6.

6. A micro-organism transformed by the plasmid of claim 3 where said micro-organism is selected from the group consisting of E.coli and Bacillus subtilis.

7. The micro-organism of claim 6, where said E.coli is Escherichia coli DH5α (pSMSRF6) ATCC 68913.

8. Use of the DNA sequence of claim 1, for the in vitro and in vivo production of surfactin and analogous molecules.
